Europäisches Patentamt

European Patent Office

Office européen des brevets

(11) Publication number: **0 108 218**
A2

# (12) EUROPEAN PATENT APPLICATION

(21) Application number: 83108974.3

(22) Date of filing: 12.09.83

(51) Int. Cl.³: **A 61 K 9/22**

(30) Priority: 08.10.82 US 433397

(43) Date of publication of application:
16.05.84 Bulletin 84/20

(84) Designated Contracting States:
AT BE CH DE FR GB IT LI LU NL SE

(71) Applicant: VEREX LABORATORIES, INC.
5241 South Quebec
Englewood Colorado 80111(US)

(72) Inventor: Dunn, James Michael
3236 Hinsdale Place
Littleton Colorado 80122(US)

(72) Inventor: Haas, Ronald Thomas
8413 S. Painted Sky Street
Highland Park Colorado 80126(US)

(74) Representative: Modiano, Guido et al,
MODIANO, JOSIF, PISANTY & STAUB Modiano &
Associati Via Meravigli, 16
I-20123 Milan(IT)

(54) Constant release rate solid oral dosage formulation of pharmaceutical compounds having a high degree of water solubility.

(57) The present invention provides constant order release tablets of drugs which have a high degree of water-solubility. The tablet formulations of this invention comprise: a therapeutically effective amount of a drug having a high degree of water solubility, from about 8-18 weight percent of an acrylic acid polymer; from about 8-18 weight percent of polyethylene glycol having a molecular weight of from 4000-6000; from about 2 to 9 weight percent of a hydrogenated vegetable oil; from about 0.5 to 3 weight percent of fumed silicon dioxide; from about 15 to 25 weight percent of a disintegrant; from about 20 to 40 weight percent of a diluent; and from 0.5 to 2 weight percent of a lubricant.

EP 0 108 218 A2

Croydon Printing Company Ltd.

-1-

CONSTANT RELEASE RATE SOLID ORAL DOSAGE
FORMULATION OF PHARMACEUTICAL COMPOUNDS
HAVING A HIGH DEGREE OF WATER SOLUBILITY

The present invention relates to improved formulations of pharmaceutically active compounds which have a high degree of water solubility such as phenylpropanolamine, chlorpheniramine, isosorbide nitrate and the like, and more specifically relates to constant release rate solid oral dosage formulations of such highly water-soluble compounds.

While it is axiomatic that an orally administered drug must first dissolve in gastrointestinal fluids in order to be absorbed into the body, medications with a high degree of water solubility present both formulation and administration problems. Such drugs are difficult to work with and care must be taken during formulation to insure that the tablets contain the proper amount of drug and that the tablets do not degrade and fall apart when exposed to 25 percent or more humidity or instantly upon being ingested. Since such highly water-soluble drugs are rapidly absorbed and hence do not exhibit a smooth onset of action or long duration of action, two-fold formulation problems exist.

Because of the undesirability of having peaks and valleys of active drug in the serum or plasma, and the desirability of providing a smoother onset of drug action with a longer duration of pharmacological activity, numerous long-acting or sustained release formulations have been marketed for a number of such products for some period of time and a vast body of prior art exists on such formulations.

However, in earlier work with drugs which are normally irritating to the gastrointestinal tract and which have undesirable side effects which generally interfere with patient compliance and hence lead to inadequate drug therapy, it was found that a constant release rate formulation has a number of advantages over conventional sustained or timed-release formulations for products such as aspirin, indomethacin, and theophylline hydrochloride. See co-pending applications Serial Nos. 366,594, filed April 8, 1982; 334,124 filed December 24, 1981; and 364,104 filed March 31, 1982 for constant release rate solid oral dosage formulations for aspirin, indomethacin and theophylline hydrochloride. See also United States Patent No. 4,308,251.

The discovery of constant order formulations which not only permit once or twice a day dosage regimens, but substantially reduce drug side effects, led to work on developing constant order release formulations for other drugs as well. It was found that in view of the widely differing solubilities and other properties of various drug entities, the solution to the problem of developing a superior, constant order solid oral dosage formulation for one drug entity did not provide a solution to the problem of providing constant release formulations for other drug entities unless, as in the present case of highly water-soluble drugs, a general solution for a class of drugs sharing similar properties is found. However, in view of the number of factors affecting successful formulation, careful experimentation is required in the development of suitable formulations for various drug entities. As discussed below, there are even a few exceptions within the class of highly water-soluble drugs which require a separate solution to the problem of providing constant order release tablets for such drug entities.

If highly water-soluble drugs are not properly formulated, the drug will go into solution and be absorbed immediately upon ingestion leading to "superbiovailability" where the patient is subjected to a slug of the active drug all at once. . This immediate release of the entire dose not only provides immediate, albeit short term drug effect, but also immediate and concentrated side effect. In the past, complicated processes and systems have been employed in formulating highly water-soluble drugs.

Such systems have included a hot wax melt system wherein melted wax is poured into the product so that the product becomes embedded in the wax; formulating individual beads coated with a release retardant agent such as ethyl cellulose and filling those beads into capsules; or puoring the drug into a plastic polymer matrix or sponge such as the Abbott Laboratories GRADUMET drug delivery system.

The present invention not only provides a superior drug formulation for such products, which formulation permits a constant, steady, slow release of drug over a 12 to 24 hour period, but also greatly simplifies the process for formulating such drugs and lowers the cost of production.

Reviewing the prior art more closely in connection with the present invention, the addition of Carbopol-934 carbomer to various molecular weights of polyethylene glycol to retard the release of active drug from a tablet is well known. See for example Poole, U.S. Patent No. 3,634,584. While Poole disclosed the use of a carbomer and polyehtylene glycol combination, Poole failed to recognize and solve the problem of sticking typically encountered when these two well known substances are used together. Nor did Poole recognize or solve the problem specifically encountered in formulating highly water-soluble drugs. In addition to Poole failing to recognize the problem solved by the present

- 4 -

invention, Poole neither discloses nor suggests the instant invention.

Similarly, the use of an acrylic acid polymer in conjuction with other polymeric materials such as polyvinyl-pyrrolidine is also known. See for example Hill U.S. Patent No. 3,458,622. However, Hill teaches away from the present invention and clearly failed to recognize the problems inherent in formulating highly water-soluble drugs. Specifically, Hill teached the use of methylene chloride as a suitable solvent. However, methylene chloride is incompatible with the formulations of the present invention, and if used, serves to form a gooey mess which cannot be readily dried and which entraps the methylene chloride in the final granuation. While Hill generally discloses suitable lubricants and binders which are employed in the present invention, Hill failed to recognize and solve the problems solved by the present invention and neither teaches nor suggests the present formulations.

The present invention provides a constant release rate tablet of drugs having a high degree of water-solubility such as phenylpropanolamine, chlorpheniramine, combinations of phenylpropanolamine and chlorpheniramine, isosorbide dinitrate and the like. The tablets of the present invention comprise an intimate admixture of active drug in combination with an acrylic acid polymer, polyethylene glycol or a suitable cellulosic derivative, a hydrogenated vegetable oil, fumed silica and a tabletting lubricant such as magnesium stearate in the critical proportions disclosed hereinbelow. The constant release tablets of the present invention are administered once or twice a day to provide the desired therapeutic effect.

FIG. 1 is a graph depicting the dissolution profile of phenylpropanolamine tablets prepared by the method of Example 1.

FIG. 2 is a graph showing the dissolution profile of phenylpropanolamine and chlorpheniramine tablets prepared by the method of Example 2.

FIG. 3 is a graph showing the dissolution profile of isosorbide dinitrate tablets prepared by the method of Example 3.

The constant order release tablets of the present invention comprise an intimate admixture of a therapeutically effective amount of a drug having a high degree of water-solubility, from about 8 to 18 weight percent and preferably from about 10 to 15 weight percent of an acrylic acid polymer; from about 8 to about 18 weight percent and preferably from about 10 to 15 weight percent of polyethylene glycol having a molecular weight of from about 4000 to 6000; from about 2 to 9 weight percent and preferably from about 4 to 8 weight percent of a hydrogenated vegetable oil; from about 0.5 to 3 weight percent of fumed silicon dioxide; from about 15 to 25 weight percent and preferably from about 18 to 22 weight percent of a disintegrant; from about 20 to about 40 weight percent of a diluent, and from 0.5 to about 2 weight percent of a suitable lubricant.

While there are a number of acrylic acid polymers which should be suitable for use in the practice of the present invention, the only acrylic acid polymer currently approved by the United States Food and Drug Administration for internal human use is a carbomer sold by B.F. Goodrich under the tradename CARBOPOL-934P. Thus, CARBOPOL-934P carbomer is the presently preferred acrylic acid polymer for use in the practice of this invention until such time as further acrylic acid polymers are approved for human internal use.

The preferred hydrogenated vegetable oil is a hydrogenated cottonseed oil sold by E. Mendell Corporation under the trademark LUBRITAB.

Magnesium stearate is the preferred lubricant, although other standard tabletting lubricants such as talc and the like can be employed in the practice of this invention.

In addition to the components listed above, a hydrophobic cellulosic binding agent may advantageously be incorporated into the formulation. The preferred cellulosic binding agent is ethyl cellulose (50cps), dispersed in isopropyl alcohol. In the event it is desired to reduce the bulk of the tablet, the polyethylene glycol may be eliminated and the cellulosic binding agent employed in greater quantity.

While a number of standard disintegrants may be employed in the practice of this invention, it is preferred to employ lactose. Similarly, while any number of standard bulking agents may be employed in the present formulations, the preferred bulking agent or diluent is dibasic calcium phosphate. Other suitable bulking agents include microcrystalline cellulose, mannitol, tricalcium phosphate, acacia, gelatin, sorbitol and methyl cellulose.

The preferred solvent employed in the process for manufacturing the tablets of the present invention is isopropyl alcohol or other suitable lower aliphatic alcohols. Methylene chloride should not be used since this will entrap both the oil and the carbomer and form a rubbery mass which cannot be dried.

It is both critical and essential to include fumed silicon dioxide (SYLOID 244) as a lubricant as well as magnesium stearate as an adjunctive lubricant to overcome the serious sticking problem encountered when tabletting highly water-soluble drugs.

The term "constant release rate", as used herein, refers to a formulation wherein the in vitro release of active drug from the tablet, using a U.S.P. dissolution apparatus, pH 7.5, is constant and linear against time untill all of the drug is released when plotted on an x,y graph using the following formula:

$$K= \frac{dc}{dt}$$

wherein K stands for constant, dc stands for decreasing concentration and dt stands for decreasing time, a straight line is formed. Calculation of at least four data points by linear regression analysis should give a value of from 0.85 to 1.0. A value of 1.0 is a perfect straight line.

The term "highly water-soluble", as used herein, refers to drugs falling within the definitions of "very soluble" or "freely soluble" on page 203 of Remington's Pharmaceutical Sciences, 16th Edition, Mack Publishing Company (1980). More specifically, the term, as used herein, refers to drugs which have a solubility of from 1 to 15 grams of drug per ml of water. It should be noted that while a number of drugs are highly soluble in other solvents, the present invention is limited to drugs which are highly soluble in water.

Included within such highly water-soluble drugs are phenylpropanolamine, chlorpheniramine, isosorbide nitrate, atropine sulfate, beta-methasone sodium phosphate, brompheniramine maleate, clindamycin phosphate, desipramine hydrochloride, dexbrompheniramine maleate, dextroamphetamine hydrochloride, dicyclomine hydrochloride, diethylproprion hydrochloride, ephedrine hydrochloride, fluroazepam, theophylline sodium oleate, tetracycline hydrochloride, morphine sulfate, pentylenetetrazole, physostigmine sulfate, scopolamine hydrobromide and wafarin potassium.

It is noted that not all salts of the same drug have the same water solubilities. For instance, while theophylline sodium oleate has a solubility of 6 grams/ml of water and may be formulated according to the present invention, theophylline hydrochloride has a solubility of 300 g/ml and is not suitable for the present formulations.

In addition, highly water soluble drugs such as propanol, quinidine and verapamil may not be formulated according to the present invention because of their pKA values and suitable formulations for these drugs are being sought.

The process for preparing the constant release rate tablets of the present invention is simple, straightforward, and results in significant savings in production costs currently encountered in the production of presently available microencapsulation, wax bead or spheroidal coated particles placed in a capsule.

For illustrative purposes, three highly water-soluble drug products, phenylpropanolamine, combination of phenylpropanolamine and chlorpheniramine and isosorbide nitrate were tabletted according to the present invention.

Each tablet of phenylpropanolamine weighed between 400-500 mg and contained 75 mg of active drug. Each tablet of phenylpropanolamine and chlorpheniramine weighed between 450-525 mg and contained 75 mg of phenylpropanolamine and 8 mg of chlorpheniramine. Tablets of isosorbide dinitrate weighed from 260 to 870 mg and contained from 50 to 160 mg of active drug.

Generally speaking, all three of these illustrative drug products are prepared in a similar manner. The active ingredient(s), carbomer, diluent, hydrogenated vegetable oil, polyethylene glycol and disintegrant are weighed and carefully mixed as a dry blend. A wet granulation is then prepared by slowing adding isopropyl alcohol, which may or may not contain dispersed ethylcellulose. The wet powder is then blended until a granule forms. The granulation is dried and reduced through a No. 16 mesh screen. The syloid and magnesium stearate may be added before granulation or added after the granulate has dried. The screened and blended granules are then compressed in a rotary press or single station tablet press.

It is important to note that compositions containing polyethylene glycol and polymers of acrylic acid in conjunction with hydrogenated vegetable oil are mechanically difficult to press into a tablet without considerable weight variation in the tablets or sticking to the tablet punches. The classical tabletting lubricants such as talc, magnesium stearate, stearic acid, cetyl alcohol, calcium stearate or zinc stearate are ineffective in alleviating this problem.

It was found that the only suitable solution to the problem was the inclusion of 0.5 to 2.0 weight percent of fumed silicon dioxide, sold under the trademark SYLOID-244 by W. Grace Company. This both alleviates the problem of tablet sticking and serves as a superior lubricant.

The polymers which form the basic tablet matix are physically bound by the addition of dibasic calcium phosphate and microcrystalline cellulose. Lactose is employed as a disintegrant. In instances where the drug is highly soluble, enhancement of the retardation effect may be achieved by the addition of a cellulosic derivative such as ethyl cellulose to enhance the retardation properties of the carbomer and polyethylene glycol matrix.

Turning to the preferred process of the present invention, all material are weighed and blended for 15 minutes for each 40 kilograms of dry material. Isopropyl alcohol which may constitute 35-60 weight percent is slowly added at a rate of 3 minutes per liter of solvent. If ethylcellulose is to be dispersed in the solvent prior to granulation, the cellulose derivative should be blended until there is complete dispersion and clarity of the solution. Generally, it will take between 35-40 minutes per 125 liters of fluid using a lightening blender to effect complete dispersion and solvation of the ethylcellulose. It is critical that the wet granulation be completely dried before screening. Failure to observe this technique may result in rupture of the granules and a loss of the constant release rate profile of the resulting tablets. In conventional prior art methods, the wet granulations are generally screened immediately after formation, then dried. If the prior art processes are employed, the constant release rate profile of the tablets may be destroyed.

Tablets made by the process described above, and illustrated in detail hereinbelow release their active drug over a specified period of time and in vitro they perform in a constant release rate manner. Because of this unique constant release rate property, the tablets of the present invention may be administered once or twice a day to provide 24 hour pharmacological activity and the desired drug effect.

The following examples further illustrate the present invention.

### Example 1

Tablets containing 75 mg of phenylpropanolamine and weighing 470 mg were prepared as follows:

| Ingredients | Amount (grams) |
|---|---|
| 1. Phenylpropanolamine hydrochloride | 75 |
| 2. Dibasic calcium phosphate | 150 |
| 3. Hydrogenated cottonseed oil | 25 |
| 4. Polyethylene glycol | 50 |
| 5. Microcrystalline cellulose | 10 |
| 6. Anhydrous lactose | 100 |
| 7. Carbopol-934P carbomer | 50 |
| 8. Fumed silicon dioxide (Syloid-244) | 6 |
| 9. Magnesium stearate | 4 |
| 10. Isopropyl alcohol | 200 |

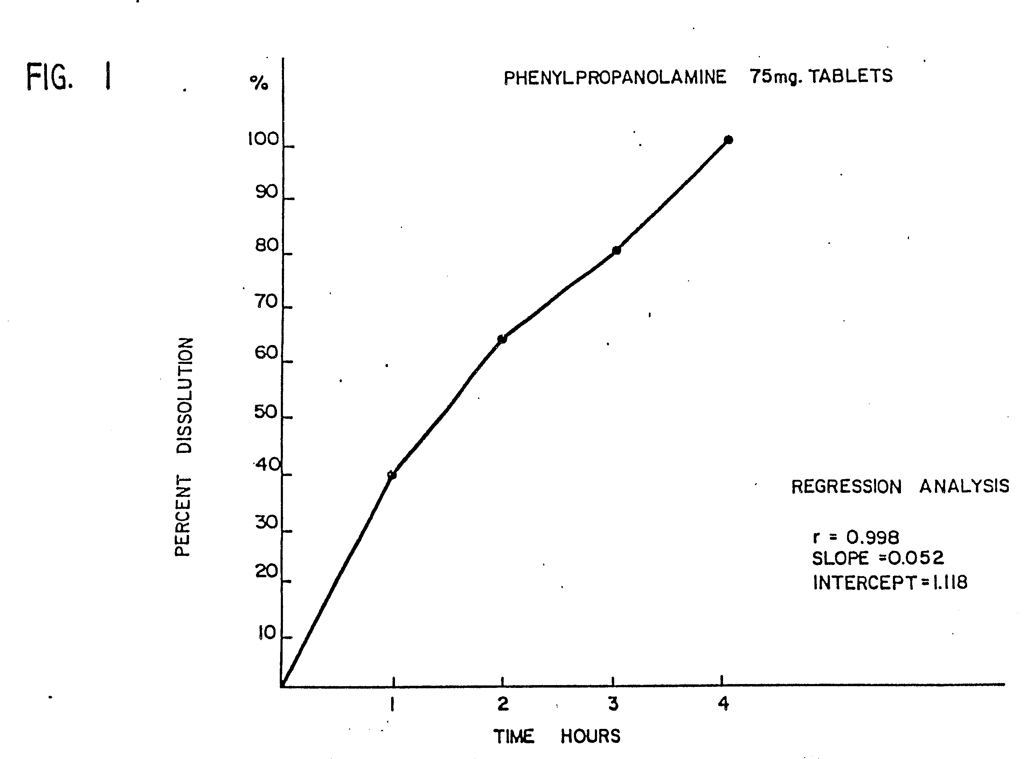

Ingredients 1-9 were weighed and placed in a Hobart blender and dry-blended. Isopropyl alcohol was added in a steady, slow stream until a wet granulation formed. The material was discharged onto paper-lined trays and dried overnight. After drying, the granules were screened through a No. 16 mesh screen and then compressed into tablets weighing 470 mg each. Each tablet contained 75 mg of phenylpropanolamine and had a hardness of 12kp. The dissolution profile of these tablets is graphically depicted in Figure 1. Regression analysis provided a value r=0.998 (slope = 0.052, intercept = 1.118). A value of 1 is a perfect straight line, and the linear regression analysis of the tablets prepared by the above example are within .002 of the optimum theoretical value 1.

## EXAMPLE 2

The following formulation was used to produce a capsule-shaped tablet weighing 480 mg and containing 75 mg each of phenylpropanolamine and 8 mg of chlorpheniramine:

| Ingredients | Amount (grams) |
|---|---|
| 1. Phenylpropanolamine hydrochloride | 75 |
| 2. Chlorpheniramine maleate | 8 |
| 3. Dibasic calcium phosphate | 150 |
| 4. Hydrogenated cottonseed oil | 25 |
| 5. Polyethylene glycol 6000 | 50 |
| 6. Microcrystalline cellulose | 10 |
| 7. Anhydrous lactose | 100 |
| 8. Carbopol 934P carbomer | 50 |
| 9. Fumed silicon dioxide | 8 |
| 10. Magnesium stearate | 4 |
| 11. Isopropyl alcohol | 200 |

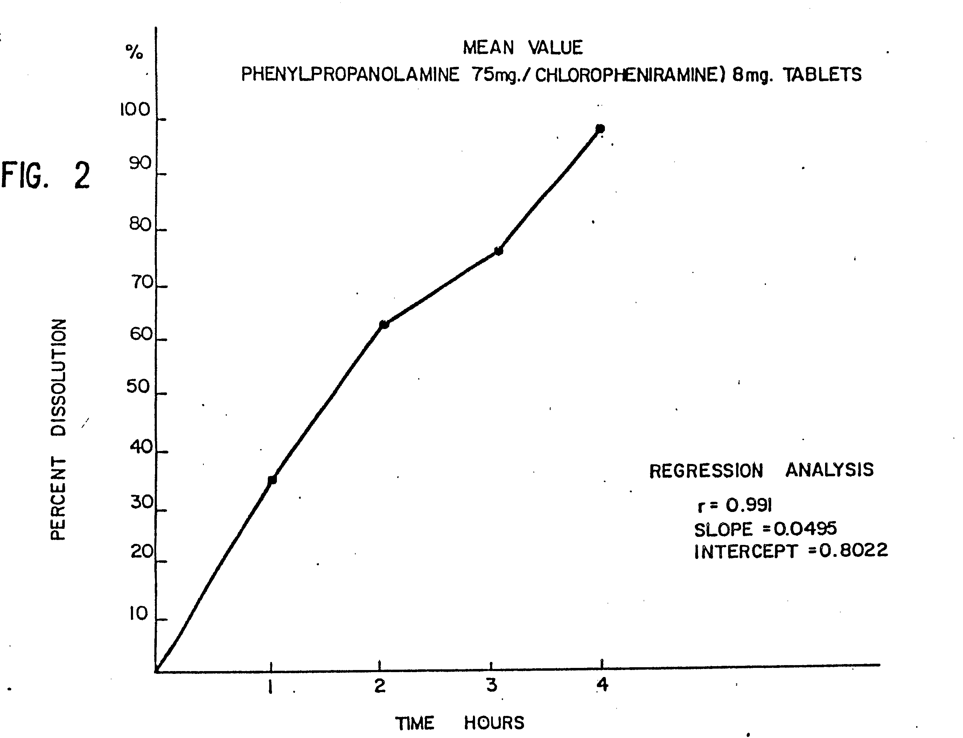

In the above formulation, ingredients 1-8 were added to a Hobart mixer and dry blended for 10 minutes. Isopropyl alcohol was added until a wet granulation formed. After complete formation of the granulate, it was discharged onto paper-lined trays and dried overnight. The granulate was then screened through No. 16 mesh screen and blended with the fumed silicon dioxide and magnesium stearate. After complete mixing, the powder was compressed into tablets weighing 480 mg each and having a hardness of 12kp. The dissolution profile of tablets prepared by this Example are shown in FIG. 2. Regression analysis of the above-prepared tablets provided a regression value r=0.991.

### EXAMPLE 3

The following formulation was used to prepare constant release rate tablets weighing 270 mg and containing 50 mg per tablet of isosorbide dinitrate. The tablets had a hardness of 10-12kp and a regression value r=0.981.

| Ingredients | Amount(grams) |
|---|---|
| 1. Isosorbide dinitrate 25% | 640 |
| 2. Polyethylene glycol 6,000 | 75 |
| 3. Carbopol-934P carbomer | 75 |
| 4. Fumed silicon dioxide | 10 |
| 5. Ethylcellulose 50 c.p.s. | 50 |
| 6. Magnesium stearate | 10 |
| 7. Isopropyl alcohol | 300ml |

Isosorbide nitrate was first diluted with lactose to provide 25 weight percent of active drug. Ingredients 1-4 were then dry-blended in a Hobart mixer. The ethyl cellulose was dissolved in 300 ml of isopropyl alcohol and the dry powder was then granulated with the ethyl cellulose-alcohol combination. After formation of the granulate, the material was discharged onto paper-lined trays and dried overnight. After drying, the granulate was screened through a No. 18 mesh screen, blended with magnesium stearate, and compressed into tablets weighing 270 mg and containing 50 mg of active drug.

## EXAMPLE 4

Isosorbide nitrate tablets were prepared from the following formulation which includes the addition of a color to the finished product.

| Ingredients | Amount(grams) |
|---|---|
| 1. Isosorbide dinitrate 25% | 640 |
| 2. Propylene glycol 6000 | 75 |
| 3. Carbopol-934P carbomer | 75 |
| 4. Fumed silicon dioxide | 10 |
| 5. Opadry yellow, non-enteric | 10 |
| 6. Ethylcellulose 50 c.p.s. | 50 |
| 7. Magnesium stearate | 10 |
| 8. Isopropyl alcohol | 300 ml |

The 25 percent isosorbide dinitrate was prepared as in Example 3, supra, by diluting the active drug with lactose. Ingredients 1-5 were then placed in a Hobart mixer and blended. After complete admixture was obtained, ethylcellulose dispersed in isopropyl alcohol was added as the granulating fluid. After formation of a wet granulation, the granulate was discharged onto paper-lined trays and dried overnight. Following complete drying of the granulate, size reduction was carried out by passing the material through a No. 16 mesh screen, blended with magnesium stearate and compressed into capsule shaped tablets weighing 870 mg and containing 160 mf of isosorbide dinitrate.

## EXAMPLE 5

Isosorbide nitrate tablets weighing 870 mg each and containing 160 mg of isosorbide nitrate were prepared from a formulation containing hydrogenated cotton seed oil to further retard dissolution.

| Ingredients | Amount (grams) |
|---|---|
| 1. Isosorbide dinitrate 25% | 640 |
| 2. Hydrogenated cottonseed oil | 50 |
| 3. Polyethylene glycol 6,000 | 50 |
| 4. Fumed silicon dioxide | 10 |
| 5. Ethyl cellulose 50 c.p.s. | 50 |
| 6. magnesium stearate | 10 |
| 8. Isopropyl alcohol | 300 ml |

Ingredients 1-5 were placed in a Hobart mixer and dry-blended. The ethylcellulose was dissolved in isopropyl alcohol and poured into the dry blend until a wet granulation was formed. The granulate was then placed on a paper-lined tray and dried overnight. After complete drying, the granulate was screened through a No. 16 mesh screen blended with the magnesium stearate and compressed into tablets having a hardness of 11-13kp.

EXAMPLE 6

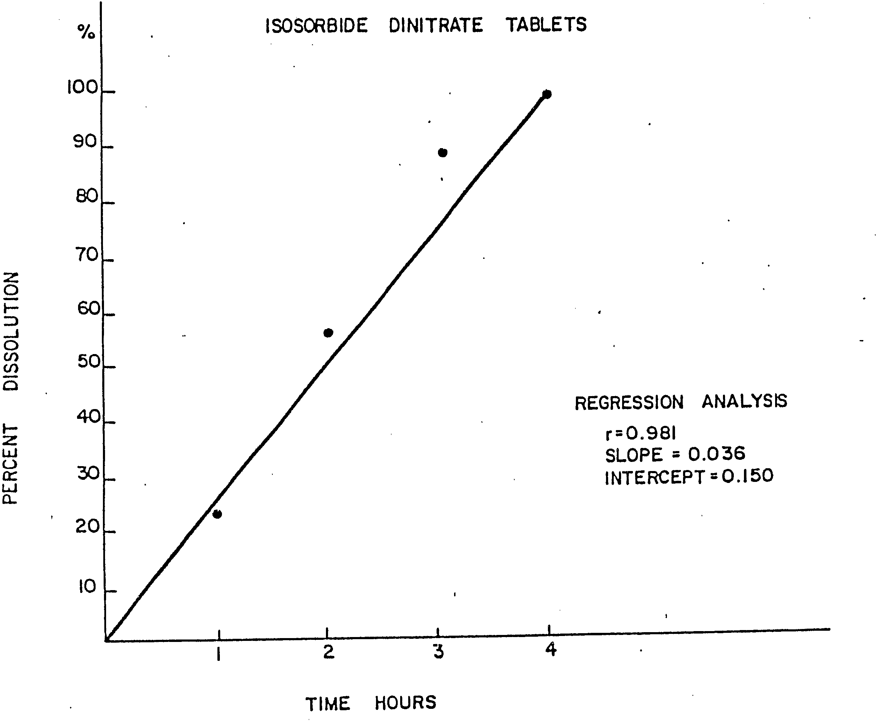

The dissolution rates of the formulations of Examples 1-3 are graphically depicted in FIGS. 1-3. The dissolution rates of the isosorbide dinitrate tablets of Examples 3 and 4, which differ only by the addition of a coloring agent, are substantially identical. The following table sets forth the _in vitro_ dissolution rate of the tabletss made by the method of Example 5. The testing methods were the same for all isosorbide nitrate formulations. Tablets were placed in an approved U.S.P. dissolution apparatus at 50rpm, pH 7.5 and 37°C and assayed for isosorbide nitrate content.

Dissolution of Isosorbide DiNitrate

Tablets of Example 5

| Tablets | 0 Hrs. | 1 Hr. | 2 Hr. | 3 Hr. | 4 Hr. |
|---|---|---|---|---|---|
| No. 1 | 96.3% | 93.6 | 91.5 | 89.2 | 86.9 |
| No. 2 | 96.0% | 94.3 | 91.0 | 88.9 | 86.5 |
| No. 3 | 101.3% | 99.0 | 96.2 | 93.8 | 90.3 |
| No. 4 | 100.8 | 99.0 | 95.8 | 93.6 | 91.0 |
| No. 5 | 98.0% | 96.3 | 93.0 | 89.8 | 88.4 |
| No. 6 | 97.9 | 96.2 | 93.0 | 91.7 | 88.4 |
| %Dissolution | 0 | 2.0% | 5.0% | 7.2% | 9.82% |

Regression analysis of these date provided the following values:

$$r = 0.998$$

$$slope = 0.388$$

$$intercept = 0.168$$

While the non-hydrogenated cottonseed oil formulation of Example 3 provided a constant release tablet (r=0.981), it is clear that the addition of a hydrogenated vegetable oil enhances the dissolution properties and it is therefore preferred to formulate tablets according to the present invention from compositions containing hydrogenated vegetable oil.

EXAMPLE 7

If smaller tablets are desired, the polyethylene glycol may be replaced with microcrystalline cellulose (Avicel) and if the ethylcellulose 50 c.p.s. is replaced with ethylcellulose 100 c.p.s. in the formulation. Tablets having less bulk that the tablets of examples 3,4 and 5 were prepared from the following formulation by the method of Example 5.

| Ingredients | Amount (grams) |
|---|---|
| 1. Isosorbide dinitrate | 240 |
| 2. Hydrogenated cottonseed oil | 200 |
| 3. Avicel | 10 |
| 4. Carbopol-934P carbomer | 100 |
| 5. Opadry yellow, non-enteric | 20 |
| 6. Fumed silicon dioxide | 50 |
| 7. Ethylcellulose 100 c.p.s | 50 |
| 8. Magnesium stearate | 10 |
| 9. Isopropyl alcohol | 300 ml |

Tablets were prepared from the above formulation employing the method of Example 5, and adding the coloring agent by the method of Example 4.

FIGS. 1-3 and Example 6 demonstrate that tablets prepared according to the present invention exhibit a constant and linear release of drug from the tablet matrix over time. As mentioned above, the addition of a hydrogenated vegetable oil substantially enhances the constant release rate properties of the tablets of this invention and considerably reduces the amount of drug released over a four-hour time period as compared to the amount of drug released over the same period from tablets prepared without the addition of this retardant agent. Thus, while it is not critical to add hydrogenated vegetable oil to the acrylic acid resin based formulations of the present invention, it is greatly preferred to do so in order to achieve the optimum results.

## EXAMPLE 8

Tablets weighing 270 mg and containing 75 mg per tablet of desipramine hydrochloride are prepared according to the method of Example 2 from the following formulation:

| Ingredient | Amount(grams) |
|---|---|
| 1. Desipramine hydrochloride | 75 |
| 2. Dibasic calcium phosphate | 150 |
| 3. Hydrogenated cottonseed oil | 30 |
| 4. Polyethylene glycol 6000 | 50 |
| 5. Microcrystalline cellulose | 10 |
| 6. Anhydrous lactose | 100 |
| 7. Carbopol-934P carbomer | 55 |
| 8. Fumed silicon dioxide | 5 |
| 9. Magnesium stearate | 4 |
| 10. Isopropyl alcohol | 200 ml |

## EXAMPLE 9

Tablets weighing 100 mg and containing 8 mg per tablet of chlorpheniramine are prepared from the following formulation by the method of Example 1:

| Ingredient | Amount(grams) |
|---|---|
| 1. Chlorpheniramine | 8 |
| 2. Dibasic calcium phosphate | 20 |
| 3. Hydrogenated cottonseed oil | 8 |
| 4. Polyethylene glycol 4000 | 15 |
| 5. Carbopol-934P carbomer | 15 |
| 6. Fumed silicon dioxide | 2 |
| 7. Magnesium stearate | 30 |
| 8. Isopropyl alchohol | 200 ml |

## EXAMPLE 10

Tablets weighing 100 mg and containing 10 mg per tablet of dextroamphetamine sulfate are prepared by the method of Example 1 from the following formulation:

| Ingredient | Amount(grams) |
|---|---|
| 1. Dextroamphetamine hyrdrochloride | 100 |
| 2. Dibasic calcium phosphate | 200 |
| 3. Hydrogenated cottonseed oil | 50 |
| 4. Polyethylene glycol 6000 | 200 |
| 5. Carbopol-934P carbomer | 200 |
| 6. Anhydrous lactose | 200 |
| 7. Fumed silicon dioxide | 30 |
| 8. Magnesium stearate | 20 |
| 9. Isopropyl alcohol | 300ml |

EXAMPLE 11

Tablets weighing 1 gram and containing 250 mg of tetracycline hydrochloride are prepared by the method of Example 1 from the following formulation:

| Ingredient | Amount (grams) |
|---|---|
| 1. Tetracycline hydrochloride | 250 |
| 2. Dibasic calcium phosphate | 150 |
| 3. Hydrogenated cottonseed oil | 80 |
| 4. Polyethylene glycol 5000 | 100 |
| 5. Carbopol-934P carbomer | 100 |
| 6. Anhydrous lactose | 220 |
| 7. Fumed silicon dioxide | 30 |
| 8. Talc | 20 |
| 9. Isopropyl alcohol | 300 ml |

The above examples have been given by way of illustration, only. It will be readily apparent to one having ordinary skill in the art that with the exception of the drugs exempted earlier, the above invention provides constant order release tablets of drugs having water solubilities of from 1 to 15 grams per ml of water.

C L A I M S:

1. A constant order release formulation of a drug having a high degree of water solubility in tablet unit dosage form, said tablet comprising: a therapeutically effective amount of a highly water-soluble drug; from about 8 to 18 weight percent of a first dissolution retardant, said dissolution retardant being an acrylic acid polymer; from about 8 to 18 weight percent of a second dissolution retardant, said second retardant selected from the group consisting of polyethylene glycol having a molecular weight of from 4000-6000, inclusive, and a cellulosic derivative; from about 15 to 25 weight percent of a disintegrant; from about 20 to 40 weight percent of a diluent; from about 0.5 to 3 weight percent of fumed silicon dioxide; and from about 0.5 to 2.0 weight percent of a lubricant.

2. The tablet of claim 1 wherein said second retardant is polyethylene glycol having a molecular weight of from 4000-6000 inclusive.

3. The tablet of claim 1 wherein said formulation additionally comprises from about 2 to 9 weight percent of a hydrogenated vegetable oil.

4. The tablet of claim 3 wherein said second retardant is polyethylene glycol having a molecular weight of from 4000-6000 inclusive.

5. The tablet of claim 4 wherein said bulking agent is dibasic calcium phosphate.

6. The tablet of claim 5 wherein said disintegrant is lactose.

7. The tablet of claim 2 wherein said acrylic acid polymer is Carbopol-934P carbomer, said hydrogenated vegetable oil is hydrogenated cottonseed oil, said disintegrant is lactose and said bulking agent is dibasic calcium phosphate.

8. The tablet of claim 7 wherein said water-soluble drug is phenylpropanolamine.

9. The tablet of claim 7 wherein said active drug is chlorpheniramine.

10. The tablet of claim 7 wherein said active drug is isosorbide dinitrate.

11. The tablet of claim 7 wherein said water-soluble drug is a combination of phenylpropanolamine and chlorpheniramine.

FIG. I

PHENYLPROPANOLAMINE 75mg. TABLETS

PERCENT DISSOLUTION (%)

TIME HOURS

REGRESSION ANALYSIS

r = 0.998
SLOPE = 0.052
INTERCEPT = 1.118

1/3

FIG. 2

MEAN VALUE
PHENYLPROPANOLAMINE 75mg./ CHLOROPHENIRAMINE) 8mg. TABLETS

REGRESSION ANALYSIS

r = 0.991
SLOPE = 0.0495
INTERCEPT = 0.8022

PERCENT DISSOLUTION

TIME HOURS

2/3

0108218

# FIG. 3

ISOSORBIDE DINITRATE TABLETS

PERCENT   DISSOLUTION

TIME HOURS

REGRESSION   ANALYSIS

r=0.981
SLOPE = 0.036
INTERCEPT = 0.150